# NEUE EUROPÄISCHE PATENTSCHRIFT

(11) **EP 3 366 352 B2**
(45) Veröffentlichungstag und Bekanntmachung des Hinweises auf die Entscheidung über den Einspruch: **24.06.2026**
(45) Hinweis auf die Patenterteilung: 29.01.2020
(21) Anmeldenummer: 18154096.4
(22) Anmeldetag: 30.01.2018
(51) Int. Cl.: A61Q 17/04, A61K 8/06, A61K 8/37

(54) **WASSERFESTES, SPRÜHBARES SONNENSCHUTZMITTEL**
WATERPROOF, SPRAYABLE SUN PROTECTION AGENT
PRODUIT DE PROTECTION SOLAIRE RÉSISTANT À L`EAU, PULVÉRISABLE

(30) Priorität: 22.02.2017 DE 102017202838
(43) Veröffentlichungstag der Anmeldung: 29.08.2018
(73) Patentinhaber: Beiersdorf AG, 22529 Hamburg (DE)
(72) Erfinder: Lerg, Heike, 22303 Hamburg (DE); Fänger, Sabine, 22763 Hamburg (DE); Gronau-Horn, Annette, 22549 Hamburg (DE); Bleckmann, Andreas, 22926 Ahrensburg (DE); Göddertz, Dominik, 22549 Hamburg (DE)
(74) Vertreter: Beiersdorf AG

(56) Entgegenhaltungen:
- DE-A1- 102015 208 872
- DATABASE GNPD [online] MINTEL; 1 February 2016 (2016-02-01), PHYSICIANS FORMULA: "Marathonista Tinted Moisturizer SPF 40", XP002782104, Database accession no. 3771205
- DERMAPHARM: "Kids Sun Lotion SPF 30", GNPD; MINTEL, 1 June 2011 (2011-06-01), XP002728717
- DATABASE GNPD [online] MINTEL; 1 August 2014 (2014-08-01), BEIERSDORF: "Mattifying Sun Fluid for Face SPF 50", XP002782105, Database accession no. 2588651
- DATABASE GNPD [online] MINTEL; 1 July 2015 (2015-07-01), STADA OCT: "Advanced Infrared-A Protection System Lotion SPF 50+", XP002782106, Database accession no. 3198803
- "Isolan GPS", 1 May 2003 (2003-05-01), pages 1 - 7, XP055135518, Retrieved from the Internet <URL:http://www.quetzalquimica.com/images/DS_ISOLAN_GPS_e19-10-2007.pdf> [retrieved on 20140820]

## Beschreibung

Die vorliegende Erfindung betrifft eine kosmetische Öl-in-Wasser-Emulsion (O/W-Emulsion) enthaltend Polyglyceryl-4 Diisostearate/Polyhydroxystearate/Sebacate in einer Menge von 0,05 bis 5 Gewichts-%, bezogen auf das Gesamtgewicht der Zubereitung, wobei die Zubereitung frei ist von 2-Ethylhexyl-2-cyano-3,3-diphenylacrylat; 4-Methoxyzimtsäure(2-ethylhexyl)ester; 4-Methoxyzimtsäureisoamylester2-Hydroxy-4-methoxybenzophenon; 2-Hydroxy-4-methoxy-4'-methylbenzophenon; 3-(4-Methylbenzyliden)campher und 3-Benzylidencampher, Propyl- und Butyl-Parabenen, Isothiazolinonen, Imidazolidinyl Urea und 3-Iodpropargyl-*N*-butylcarbamat (IPBC).

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Eine besondere Form kosmetischer Sonnenschutzmittel stellen die sprühbare Zubereitungen dar, die mit Hilfe eines Sprühapplikators mit Sprühkopf entweder mit Hilfe einer mechanischen Förderpumpe oder mittels eines Treibgases aus einem Vorratsbehältnis auf die Haut appliziert werden. An derartige Zubereitungen werden eine Reihe von Anforderungen gestellt, die in ihrer Gesamtheit oft nur schwer zu vereinbaren sind.

So sollen die Zubereitungen einen guten UV-Schutz gewährleisten, sich dabei angenehm auf der Haut anfühlen, gut zu versprühen sein (d.h. leicht aus dem Vorratsbehälter zu entnehmen sein und sich dabei gelichmäßig, großflächig auf der Haut verteilen) und dabei nicht so dünnflüssig sein, dass sie nach den Auftragen auf die Haut sofort herunterfließen, sondern ein gewisses "Haftungsvermögen" auf der Haut aufzeigen, um anschließend wiederum einfach und gleichmäßig auf der Haut zu verteilen sein.

Meist verwendet man zur Erreichung dieser Eigenschaften Zubereitungen auf der Basis von Öl-in-Wasser-Emulsionen (O/W-Emulsionen). Dabei bereitet es jedoch Schwierigkeiten, dass die Zubereitungen nicht "wasserfest" sind, da ihre äußere Phase ja aus Wasser gebildet wird. Die so genannte "Wasserfestigkeit" von Sonnenschutzmitteln auf der Haut ist jedoch eine wesentliche Voraussetzung für einen effektiven UV-Schutz, wenn Sonnenschutzmittel im Zusammenhang mit Wassersport (Schwimmen, Surfen, Tauchen etc.) zum Einsatz kommen. Es gibt daher einen großen Bedarf an "wasserfesten" Sonnenschutzmitteln.

Um die "Wasserfestigkeit" von Sonnenschutzmitteln zu erhöhen, werden diesen üblicherweise sogenannte Filmbildner zugesetzt, welche die UV-Filter durch einen durch sie gebildeten Polymerfilm auf der Haut fixieren sollen. Diese Filmbildner haben jedoch Nachteile im Hinblick auf das durch sie hervorgerufene Hautgefühl, dass als "schwer" und "klebrig" beschrieben wird und, beispielsweise bei der Anwendung am Strand, zu einer verstärkten Sandanhaftung der Produkte führt.

DE102018208872 offenbart Octocrylen-freie Sonnenschutzmittel auf Basis von O/W-Emulsionen.

Es war daher die Aufgabe der vorliegenden Erfindung, die Nachteile des Standes der Technik zu vermeiden und einen Weg zu finden, sensorisch attraktive sprühbare O/W-Emulsionen (insbesondere Sonnenschutzmittel) herstellen zu können, die, auf die Haut aufgetragen, eine hohe Wasserfestigkeit aufweisen.

Überraschend gelöst wird die Aufgabe durch eine kosmetische Öl-in-Wasser-Emulsion (O/W-Emulsion) gemäß Anspruch 1, 13 und 24.

Dabei ist es erfindungsgemäß bevorzugt, wenn die Emulsion Polyglyceryl-4 Diisostearate/Polyhydroxystearate/Sebacate in einer Menge von 0,1 bis 2 Gewichts-%, bezogen auf das Gesamtgewicht der Zubereitung, enthält.

Diisostearate/Polyhydroxystearate/Sebacate kann beispielsweise unter dem Handelsnamen "Isolan GPS" bei der Firma Evonik bezogen werden. Dieser Emulgator hat einen HLB-Wert von etwa 5 und ist daher ein typischer W/O-Emulgator. Überraschend an der vorliegenden Erfindung war insbesondere, dass sich dieser W/O-Emulgator stabil und ohne Phasenumkehr in O/W-Emulsionen einarbeiten lässt und, trotz seiner gewissen Affinität zu Wasser, die Wasserfestigkeit der Zubereitung erhöht.

Erfindungsgemäß enthält die erfindungsgemäße Zubereitung einen oder mehrere UV-Filter.

So ist es erfindungsgemäß von Vorteil, wenn die Zubereitung gemäß Anspruch 13 und 24 2-Ethylhexyl 2-hydroxybenzoat (INCI: Ethylhexyl Salicylate) und/oder 3,3,5-Trimethylcyclohexyl 2-hydroxybenzoat (INCI: Homosalate) enthält.

Enthält die Emulsion 2-Ethylhexyl 2-hydroxybenzoat (INCI: Ethylhexyl Salicylate), so beträgt die erfindungsgemäß vorteilhafte Einsatzkonzentration von 0,5 bis Gewichts 5 %, bezogen auf das Gesamtgewicht der Zubereitung.

Enthält die Emulsion 3,3,5-Trimethylcyclohexyl 2-hydroxybenzoat (INCI: Homosalate), so beträgt die erfindungsgemäß vorteilhafte Einsatzkonzentration von 0,5 bis Gewichts10 %, bezogen auf das Gesamtgewicht der Zubereitung.

Darüber hinaus ist es Vorteilhaft im Sinne der vorliegenden Erfindung, wenn die Zubereitung gemäß Anspruch 1 und 24 2-Phenylbenzimidazol-5-sulfonsäuresalze enthält. Dabei wird bevorzugt das Natriumsalz eingesetzt. Dieses kann beispielsweise durch Zusatz von Natronlauge in eine 2-Phenylbenzimidazol-5-sulfonsäure enthaltende Zubereitung gebildet werden.

Enthält die Emulsion 2-Phenylbenzimidazol-5-sulfonsäuresalze, so beträgt die erfindungsgemäß vorteilhafte Einsatzkonzentration von 0,1 bis 2 Gewichts-%, bezogen auf das Gesamtgewicht der Zubereitung.

Ferner sind erfindungsgemäß vorteilhafte Ausführungsformen der vorliegenden Erfindung dadurch gekennzeichnet, dass die Emulsion gemäß Anspruch 1 und 13 einen oder mehrere UV-Filter gewählt aus der Gruppe der Verbindungen Hexyl 2-[4-(diethylamino)-2-hydroxybenzoyl]benzoate (INCI: Diethylamino hydroxybenzoyl hexyl benzoate), 4-(tert.-Butyl)-4'-methoxydibenzoylmethan, 2,4,6-Tris-[anilino-(p-carbo-2'-ethyl-1'-hexyloxy)]-1,3,5-triazin (INCI: Ethylhexyl Triazone), 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine), 4,4'-[[6-[[4-[[(1,1 dimethylethyl)amino]carbonyl]phenyl]amino]-1,3,5-triazine-2,4-diyl]diimino]bis-,(2-ethylhexyl)benzoat (INCI: Diethylhexyl Butamido Triazone), Titandioxid enthält.

Hexyl 2-[4-(diethylamino)-2-hydroxybenzoyl]benzoate (INCI: Diethylamino hydroxybenzoyl hexyl benzoate) kann dabei erfindungsgemäß vorteilhaft in einer Konzentration von 0,1 bis 5 Gewichts-%, bezogen auf das Gesamtgewicht der Zubereitung eingesetzt werden.

4-(tert.-Butyl)-4'-methoxydibenzoylmethan kann dabei erfindungsgemäß vorteilhaft in einer Konzentration von 0,5 bis 5 Gewichts-%, bezogen auf das Gesamtgewicht der Zubereitung eingesetzt werden.

2,4,6-Tris-[anilino-(p-carbo-2'-ethyl-1'-hexyloxy)]-1,3,5-triazin (INCI: Ethylhexyl Triazone) kann dabei erfindungsgemäß vorteilhaft in einer Konzentration von 0,5 bis 5 Gewichts-%, bezogen auf das Gesamtgewicht der Zubereitung eingesetzt werden.

2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine) kann dabei erfindungsgemäß vorteilhaft in einer Konzentration von 0,2 bis 5 Gewichts-%, bezogen auf das Gesamtgewicht der Zubereitung eingesetzt werden.

Diethylhexyl Butamido Triazone) kann dabei erfindungsgemäß vorteilhaft in einer Konzentration von 0,1 bis 4 Gewichts-%, bezogen auf das Gesamtgewicht der Zubereitung eingesetzt werden.

Titandioxid kann dabei erfindungsgemäß vorteilhaft in einer Konzentration von 0,1 bis 4 Gewichts-%, bezogen auf das Gesamtgewicht der Zubereitung eingesetzt werden.

Nicht zuletzt um sprühbar zu sein ist es erfindungsgemäß vorteilhaft, wenn die erfindungsgemäße Emulsion eine Viskosität von 300 bis 3000 mPas.

Die im Rahmen der vorliegenden Schrift aufgeführten Viskositätswerte der Zubereitungen und Einzelsubstanzen wurden mit Hilfe eines Viskosimeters des Typs Rheomat 123 von pro Rheo mit dem Messkörper 1 durchgefürt.

Es ist erfindungsgemäß von Vorteil, wenn die Emulsion Xanthangummi (Xanthan gum) und/oder Zellulosegummi (Cellulose gum) enthält.

Enthält die erfindungsgemäße Emulsion Xanthangummi, so beträgt die vorteilhafte Einsatzkonzentration von 0,01 bis 2 Gewichts-%, bezogen auf das Gesamtgewicht der Zubereitung.

Enthält die erfindungsgemäße Emulsion Zellulosegummi, so beträgt die vorteilhafte Einsatzkonzentration von 0,01 bis 2 Gewichts-%, bezogen auf das Gesamtgewicht der Zubereitung.

Ferner ist es erfindungsgemäß von Vorteil, wenn die Emulsion mikrokristalline Zellulose in Kombination mit Cellulose Gum enthält. Die erfindungsgemäß vorteilhafte Einsatzkonzentration beträgt von 0,01 bis 2 Gewichts-%, bezogen auf das Gesamtgewicht der Zubereitung.

Üblicherweise wird die erfindungsgemäße Emulsion mit Hilfe eines oder mehrerer O/W-Emulgatoren gebildet. Vorteilhaft im Sinne der vorliegenden Erfindung ist es, wenn ein oder mehrere Emulgatoren gewählt werdn aus der Gruppe der Verbindungen Polyglyceryl-10 stearat, Glycerylstearatcitrat, Glycerylstearat (selbstemulgierend), Polyglyceryl-3-methylglycosedistearat, Natriumcetearylsulfat, Kaliumcetylphosphat, Natriumstearoylglutamat, Sucrosepolystearat.

Dabei ist es erfindungsgemäß bevorzugt, wenn als Emulgatoren Natriumstearoylglutamat und/oder Sucrosepolystearat enthält, wobei die Kombination aus Natriumstearoylglutamat und Sucrosepolystearat in Kombination mit Hydrogenated Polyisobutene besonders bevorzugt wird.

Üblicherweise werden der oder die Emulgatoren in einer Konzentration von 0,05 bis 5 Gewichts-%, bezogen auf das Gesamtgewicht der Emulsion eingesetzt.

Bei der erfindungsgemäß besonders bevorzugten Kombination aus Natriumstearoylglutamat und Sucrosepolystearat, beträgt die bevorzugte Konzentration für Natriumstearoylglutamat von 0,1bis 2 Gewichts-%, und für Sucrosepolystearat in Kombination mit Hydrogenated Polyisobutene von 0,1 bis 2 Gewichts-%, jeweils bezogen auf das Gesamtgewicht der Emulsion.

Erfindungsgemäß besonders bevorzugt ist die erfindungsgemäße Emulsion frei ist von Polyethylenglycol-Ethern und Polyethylenglycol-Estern, d.h. "PEG-frei".

Es ist erfindungsgemäß vorteilhaft, wenn die erfindungsgemäße Emulsion einen oder mehrere Wirkstoffe gewählt aus der Gruppe der Verbindungen Glycyrrhetinsäure, Harnstoff, Arctiin, alpha-Liponsäure, Folsäure, Phytoen, D-Biotin, Coenzym Q10, Hyaluronsäure, alpha-Glucosylrutin, Carnitin, Carnosin, Coffein, natürliche und/oder synthetische Isoflavonoide, Glycerylglucose, Kreatin, Kreatinin, Taurin, β-Alanin und/oder Licochalcon A, Panthenol, Tocopherol, Tocopherolacetat, Vitamin C, Vitamin C Derivate, Glycyrrhiza Inflata Root Extract, Magnolienextrakt enthält.

Außerdem sind erfindungsgemäß vorteilhafte Ausführungsformen der vorliegenden Erfindung dadurch gekennzeichnet, dass die Zubereitung Propylenglycol, Butylenglycol, 2-Methylpropan-1,3-diol, Ethylhexylglycerin, 1,2-Pentandiol, 1,2-Hexandiol, 1,2-Octandiol und/oder 1,2-Decandiol enthält. Dabei ist der Gehalt an Ethylhexylglycerin erfindungsgemäß bevorzugt. Enthält die erfindungsgemäße Emulsion Ethylhexylglycerin, so ist es erfindungsgemäß von Vorteil, diese Verbindung in einer Konzentration von 0,05 bis 1,5 Gewichts-%, bezogen auf das Gesamtgewicht der Emulsion einzusetzen.

Vorteilhaft kann die erfindungsgemäße Emulsion auch einen oder mehrere Parfümstoffe enthalten. Dabei ist es erfindungsgemäß von Vorteil, wenn einer oder mehrere der folgenden Parfümstoffe in der Zubereitung enthalten ist: Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Evernia Furfuracea Extract, Evernia Prunastri Extract, Ethylenbrassylat, Farnesol, Guajakholzöl, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin.

Erfindungsgemäß vorteilhaft ist es, wenn die erfindungsgemäße Emulsion Vinylpyrrolidon/Hexadecen-Copolymer enthält. Die erfindungsgemäß vorteilhafte Einsatzkonzentration für dieses Coplymer beträgt von 0,1 bis 3 Gewichts-%, bezogen auf das Gesamtgewicht der Emulsion.

Erfindungsgemäß von Vorteil ist es auch, wenn die erfindungsgemäße Emulsion Iminodisuccinat-Salze enthält. Dabei wird bevorzugt das Tetra-Natriumsalz verwendet.

Die erfindungsgemäß vorteilhafte Einsatzkonzentration für dieses Salz beträgt von 0,05 bis 2 Gewichts-%, bezogen auf das Gesamtgewicht der Emulsion.

Die Wasserphase der erfindungsgemäßen Zubereitungen kann vorteilhaft übliche kosmetische Hilfsstoffe enthalten, wie beispielsweise Isopropanol oder Polyole niedriger C-Zahl sowie deren Ether, vorzugsweise Propylenglykol, Glycerin, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON).

Die erfindungsgemäße Zubereitung kann vorteilhaft Feuchthaltemittel enthalten. Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel^{®}1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9) und /oder Talkum und/oder Polyethylen, Nylon, Silica Diemthyl Silyate.

Es ist erfindungsgemäß bevorzugt, wenn die erfindungsgemäße Zubereitung Silica Dimethyl Silylate enthält.

Erfindungsgemäß vorteilhafte Ausführungsformen der vorliegenden Erfindung sind dadurch gekennzeichnet, dass die Zubereitung ein oder mehrere Öle gewählt aus der Gruppe der Verbindungen Butylene Glycol Dicaprylat/Dicaprat, Phenethyl Benzoat, C12-15 Alkyl Benzoat, Dibutyladipat; Diisopropylsebacate, Dicaprylylcarbonat, Di-C12-13 Alkyl Tartrate, Butyloctyl Salicylate, Diethylhexyl Syringylidene Malonate, Hydragenated Castor Oil Dimerate, Triheptanoin, C12-13 Alkyl Lactate, C16-17 Alkyl Benzoate, Propylheptyl Caprylate, Caprylic/Capric Triglyceride, Diethylhexyl 2,6-Naphthalate, Octyldodecanol, Caprylic/Capric Triglyceride, Ethylhexyl Cocoate, enthält.

Außerdem ist es erfindungsgemäß von Vorteil, wenn die Zubereitung Wachse aus der Gruppe von Copernicia Cerifera Cera,C18-36 Acid Triglyceride, C18-38 Alkyl Hydroxystearoyl Stearate, Synthetic Beeswax, Hydrogenated Coco-Glycerides und Synthetic Wax enthält.

Dabei ist es erfindugnsgemäß bevorzugt, wenn die Zubereitung Dibutyladipat, Dicaprylylcarbonat und/oder C12-C15 Alkylbenzoat enthält.

Erfindungsgemäß ist auch die Verwendung von Polyglyceryl-4 Diisostearate/Polyhydroxystearate/Sebacate zur Erhöhung der Wasserfestigkeit von O/W-Emulsionen.

Die erfindungsgemäße Wasserfestigkeit nach der folgenden Methode bestimmt:
Colipa guideline for evaluating sun product water resistance. International Method COLIPA - CTFA SA - JCIA - CTFA 2006 / International Standard ISO 24444:2010(E)

Erfindungsgemäß ist auch die Verwendung von Polyglyceryl-4 Diisostearate/Polyhydroxystearate/Sebacate zur Erhöhung des Lichtschutzfaktors (SPF) von O/W-Emulsionen.

**Vergleichsversuch**

| inci | Ansatz 1 | Ansatz 2 |
|---|---|---|
| Ethylhexylglycerin | 0,3 | 0,3 |
| Dibutyl Adipate | 2 | 2 |
| Copernicia Cerifera Cera | 1,5 | 1,5 |
| Sucrose Polystearate + Hydrogenated Polyisobutene | 1 | 1 |
| Sodium Stearoyl Glutamate | 0,4 | 0,4 |
| **Polyglyceryl-4 Diisostearate/ Polyhydroxystearate/Sebacate** | **0** | **0,5** |
| VP/Hexadecene Copolymer | 1 | 1 |
| Parfum | 0,52 | 0,5 |
| Glycerin + Aqua | 1 | 1 |
| Aqua + Sodium Hydroxide | 0,05 | 0,05 |
| Phenoxyethanol | 0,5 | 0,5 |
| Cellulose Gum | 0,5 | 0,5 |
| Acrylates/C10-30 Alkyl Acrylate Crosspolymer | 0,1 | 0,1 |
| Xanthan Gum | 0,12 | 0,12 |
| Microcrystalline Cellulose | 1 | 1 |
| Aqua | ad 100 | ad 100 |
| Alcohol Denat. + Aqua | 5 | 5 |
| Aqua + Trisodium EDTA | 1 | 1 |
| Tetrasodium Iminodisuccinate | 0,75 | 0,75 |
| Homosalate | 9 | 9 |
| Ethylhexyl Salicylate | 4,75 | 4,75 |
| Bis-Ethylhexyloxyphenol Methoxyphenyl Triazine | 3 | 3 |
| Ethylhexyl Triazone | 3 | 3 |
| Butyl Methoxydibenzoylmethane | 4,75 | 4,75 |
| Phenylbenzimidazole Sulfonic Acid | 0,5 | 0,5 |
| | | |
| Wasserfestigkeit | 46% | 58% |
| SPF | 57 | 65 |

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| | A | B | C | D | E |
|---|---|---|---|---|---|
| Copernicia Cerifera Cera | 1 | | | | |
| C18-36 Acid Triglyceride | | 1,5 | | | |
| C18-38 Alkyl Hydroxystearoyl Stearate | | | 0,5 | | |
| Synthetic Beeswax | | | | 0,5 | |
| Hydrogenated Coco-Glycerides | | | | | 0,5 |
| Synthetic Wax | | | | | 1 |
| Polyglyceryl-4 Diisostearate/Polyhydroxystearate/Sebacate | 0,25 | 0,5 | 0,75 | 0,25 | 1,5 |
| Polyglyceryl-10 Stearate | | | | | 0,5 |
| Polyglyceryl-3 Methylglucose Distearate | | | 0,3 | | |
| Glyceryl Stearate SE | | 1 | | | |
| Glyceryl Stearate Citrate | | | | 2 | |
| Sodium Cetearyl Sulfate | | 0,15 | | | |
| Sodium Stearoyl Glutamate | 0,4 | | | | |
| Sucrose Polystearate in Kombination mit Hydrogenated Polyisobutene | 1 | | | | |
| Potassium Cetyl Phosphate | | | | | |
| VP/Hexadecene Copolymer | 1 | 0,5 | 0,1 | 0,5 | |
| Xanthan Gum | 0,1 | 0,3 | 0,3 | 0,15 | 0,3 |
| Cellulose Gum | 0,5 | 0,25 | 0,25 | 0,75 | 1 |
| Microcrystalline Cellulose + Cellulose Gum | 1 | 0,5 | 0,5 | | 0,75 |
| Acrylates/C10-30 Alkyl Acrylate Crosspolymer | 0,1 | | | | 0,3 |
| Diethylamino Hydroxybenzoyl Hexyl Benzoate | | 0,5 | | 0,5 | |
| Butyl Methoxydibenzoylmethane | 3,5 | 5 | 4,5 | 3 | 4 |
| Ethylhexyl Salicylate | 4,5 | | 0,5 | 4,5 | |
| Homosalate | 9 | 9 | | 9 | |
| Phenylbenzimidazole Sulfonic Acid | 1 | 0,5 | 1,5 | | 0,5 |
| Ethylhexyl Triazone | 2 | | 3 | 2 | 2 |
| Bis-Ethylhexyloxyphenol Methoxyphenyl Triazine | 3,5 | 4 | 3 | 3 | 3,5 |
| Diethylhexyl Butamido Triazone | | | 0,5 | | |
| Titanium Dioxide | | 2 | | | 0,5 |
| Ethylhexylglycerin | 0,5 | 0,25 | 0,1 | | |
| Aqua + Trisodium EDTA | 1 | 0,5 | 1 | 0,5 | 1 |
| Phenoxyethanol | 0,5 | 0,5 | 0,3 | 0,1 | |
| Alcohol Denat. + Aqua | 4 | 4 | 5 | 6 | 7 |
| Tetrasodium Iminodisuccinate | 0,2 | 0,4 | 0,1 | 0,5 | 0,1 |
| Glycerin + Aqua | 5 | 9 | 3 | 4 | 7 |
| Dibutyl Adipate | 1 | 2 | 2 | | 1,5 |
| C12-15 Alkyl Benzoate | 4 | | 2 | 2 | 1,5 |
| Butylene Glycol Dicaprylate/Dicaprate | | 3 | 1,5 | 2 | 1,5 |
| Aqua + Sodium Hydroxide | 0,41 | 0,05 | 0,495 | | 0,46 |
| Parfum | 0,3 | 0,2 | 0,5 | 0,4 | 0,3 |
| Aqua | ad 100 | ad 100 | ad 100 | ad 100 | ad 100 |

## Patentansprüche

1. Kosmetische Öl-in-Wasser-Emulsion (O/W-Emulsion) enthaltend Polyglyceryl-4 Diisostearate/Polyhydroxystearate/Sebacate in einer Menge von 0,05 bis Gewichts-5 %, bezogen auf das Gesamtgewicht der Zubereitung,
wobei die Zubereitung frei ist von 2-Ethylhexyl-2-cyano-3,3-diphenylacrylat; 4-Methoxyzimtsäure(2-ethylhexyl)ester; 4-Methoxyzimtsäureisoamylester2-Hydroxy-4-methoxybenzophenon; 2-Hydroxy-4-methoxy-4'-methylbenzophenon; 3-(4-Methylbenzyliden)campher und 3-Benzylidencampher, Propyl- und Butyl-Parabenen, Isothiazolinonen, Imidazolidinyl Urea und 3-Iodpropargyl-N-butylcarbamat (IPBC), **dadurch gekennzeichnet, dass** die Zubereitung 2-Ethylhexyl 2-hydroxybenzoat (INCI: Ethylhexyl Salicylate) und/oder 3,3,5-Trimethylcyclohexyl 2-hydroxybenzoat (INCI: Homosalate) enthält.

2. Kosmetische O/W-Emulsion nach Anspruch 1, **dadurch gekennzeichnet, dass** die Zubereitung 2-Phenylbenzimidazol-5-sulfonsäuresalze enthält.

3. Kosmetische O/W-Emulsion nach einem der vorhergehenden Ansprüche 1 und 2, **dadurch gekennzeichnet, dass** die Emulsion einen oder mehrere UV-Filter gewählt aus der Gruppe der Verbindungen Hexyl 2-[4-(diethylamino)-2-hydroxybenzoyl]benzoate (INCI: Diethylamino hydroxybenzoyl hexyl benzoate), 4-(tert.-Butyl)-4'-methoxydibenzoylmethan, 2,4,6-Tris-[anilino-(p-carbo-2'-ethyl-1'-hexyl-oxy)]-1,3,5-triazin (INCI: Ethylhexyl Triazone), 2,4-Bis-{[4-(2-ethyl-hexyloxy)-2-hydro-xy]-phenyl}-6-(4-methoxyphenyl)-1,3,5-triazin (INCI: Bis- Ethylhexyloxyphenol methoxyphenyl Triazine), 4,4'-[[6-[[4-[[(1,1 dimethylethyl)amino]carbonyl]phenyl] amino]-1,3,5-triazine-2,4-diyl]diimino]bis-, bis(2-ethylhexyl)benzoat (INCI: Diethylhexyl Butamido Triazone), Titandioxid enthält.

4. Kosmetische O/W-Emulsion nach einem der vorhergehenden Ansprüche 1 bis 3, **dadurch gekennzeichnet, dass** die Emulsion eine Viskosität von 300 bis 3000 mPas aufweist, gemessen mit dem Viskosimeter des Typs Rheomat 123 von pro Rheo mit dem Messkörper 1 durchgeführt.

5. Kosmetische O/W-Emulsion nach einem der vorhergehenden Ansprüche 1 bis 4, **dadurch gekennzeichnet, dass** die Emulsion Xanthangummi (Xanthan gum) und/oder Zellulosegummi (Cellulose gum) enthält.

6. Kosmetische O/W-Emulsion nach einem der vorhergehenden Ansprüche 1 bis 5, **dadurch gekennzeichnet, dass** die Emulsion mikrokristalline Zellulose enthält.

7. Kosmetische O/W-Emulsion nach einem der vorhergehenden Ansprüche 1 bis 6, **dadurch gekennzeichnet, dass** die Emulsion einen oder mehrere Emulgatoren gewählt aus der Gruppe der Verbindungen Polyglyceryl-10 stearat, Glycerylstearatcitrat, Glycerylstearat (selbstemulgierend), Polyglyceryl-3-methylglycosedistearat, Natriumcetearylsulfat, Kaliumcetylphosphat, Natriumstearoylglutamat, Sucrosepolystearat enthält.

8. Kosmetische O/W-Emulsion nach einem der vorhergehenden Ansprüche 1 bis 7, **dadurch gekennzeichnet, dass** die Emulsion als Emulgatoren Natriumstearoylglutamat und/oder Sucrosepolystearat enthält.

9. Kosmetische O/W-Emulsion nach einem der vorhergehenden Ansprüche 1 bis 8, **dadurch gekennzeichnet, dass** die Emulsion frei ist von Polyethylenglycol-Ethern und Polyethylenglycol-Estern.

10. Kosmetische O/W-Emulsion nach einem der vorhergehenden Ansprüche 1 bis 9, **dadurch gekennzeichnet, dass** die Emulsion Ethylhexylglycerin enthält.

11. Kosmetische O/W-Emulsion nach einem der vorhergehenden Ansprüche 1 bis 10, **dadurch gekennzeichnet, dass** die Emulsion Vinylpyrrolidon/Hexadecen-Copolymer enthält.

12. Kosmetische O/W-Emulsion nach einem der vorhergehenden Ansprüche 1 bis 11, **dadurch gekennzeichnet, dass** die Emulsion Iminodisuccinat-Salze enthält.

13. Kosmetische Öl-in-Wasser-Emulsion (O/W-Emulsion) enthaltend Polyglyceryl-4 Diisostearate/Polyhydroxystearate/Sebacate in einer Menge von 0,05 bis Gewichts-5 %, bezogen auf das Gesamtgewicht der Zubereitung,
wobei die Zubereitung frei ist von 2-Ethylhexyl-2-cyano-3,3-diphenylacrylat; 4-Methoxyzimtsäure(2-ethylhexyl)ester; 4-Methoxyzimtsäureisoamylester2-Hydroxy-4-methoxybenzophenon; 2-Hydroxy-4-methoxy-4'-methylbenzophenon; 3-(4-Methylbenzyliden)campher und 3-Benzylidencampher, Propyl- und Butyl-Parabenen, Isothiazolinonen, Imidazolidinyl Urea und 3-Iodpropargyl-N-butylcarbamat (IPBC), **dadurch gekennzeichnet, dass** die Zubereitung 2-Phenylbenzimidazol-5-sulfonsäuresalze enthält.

14. Kosmetische O/W-Emulsion nach Anspruch 13, **dadurch gekennzeichnet, dass** die Emulsion einen oder mehrere UV-Filter gewählt aus der Gruppe der Verbindungen Hexyl 2-[4-(diethylamino)-2-hydroxybenzoyl]benzoate (INCI: Diethylamino hydroxybenzoyl hexyl benzoate), 4-(tert.-Butyl)-4'-methoxydibenzoylmethan, 2,4,6-Tris-[anilino-(p-carbo-2'-ethyl-1'-hexyloxy)]-1,3,5-triazin (INCI: Ethylhexyl Triazone), 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine), 4,4'-[[6-[[4-[[(1,1 dimethylethyl)amino]carbonyl]phenyl] amino]-1,3,5-triazine-2,4-diyl]diimino]bis-, bis(2-ethylhexyl)benzoat (INCI: Diethylhexyl Butamido Triazone), Titandioxid enthält.

15. Kosmetische O/W-Emulsion nach einem der vorhergehenden Ansprüche 13 bis 14, **dadurch gekennzeichnet, dass** die Emulsion eine Viskosität von 300 bis 3000 mPas aufweist, gemessen mit Viskosimeter des Typs Rheomat 123 von pro Rheo mit dem Messkörper 1 durchgeführt.

16. Kosmetische O/W-Emulsion nach einem der vorhergehenden Ansprüche 13 bis 15, **dadurch gekennzeichnet, dass** die Emulsion Xanthangummi (Xanthan gum) und/oder Zellulosegummi (Cellulose gum) enthält.

17. Kosmetische O/W-Emulsion nach einem der vorhergehenden Ansprüche 13 bis 16, **dadurch gekennzeichnet, dass** die Emulsion mikrokristalline Zellulose enthält.

18. Kosmetische O/W-Emulsion nach einem der vorhergehenden Ansprüche 13 bis 17, **dadurch gekennzeichnet, dass** die Emulsion einen oder mehrere Emulgatoren gewählt aus der Gruppe der Verbindungen Polyglyceryl-10 stearat, Glycerylstearatcitrat, Glycerylstearat (selbstemulgierend), Polyglyceryl-3-methylglycosedistearat, Natriumcetearylsulfat, Kaliumcetylphosphat, Natriumstearoylglutamat, Sucrosepolystearat enthält.

19. Kosmetische O/W-Emulsion nach einem der vorhergehenden Ansprüche 13 bis 18, **dadurch gekennzeichnet, dass** die Emulsion als Emulgatoren Natriumstearoylglutamat und/oder Sucrosepolystearat enthält.

20. Kosmetische O/W-Emulsion nach einem der vorhergehenden Ansprüche 13 bis 19, **dadurch gekennzeichnet, dass** die Emulsion frei ist von Polyethylenglycol-Ethern und Polyethylenglycol-Estern.

21. Kosmetische O/W-Emulsion nach einem der vorhergehenden Ansprüche 13 bis 20, **dadurch gekennzeichnet, dass** die Emulsion Ethylhexylglycerin enthält.

22. Kosmetische O/W-Emulsion nach einem der vorhergehenden Ansprüche 13 bis 21, **dadurch gekennzeichnet, dass** die Emulsion Vinylpyrrolidon/Hexadecen-Copolymer enthält.

23. Kosmetische O/W-Emulsion nach einem der vorhergehenden Ansprüche 13 bis 22, **dadurch gekennzeichnet, dass** die Emulsion Iminodisuccinat-Salze enthält.

24. Kosmetische Öl-in-Wasser-Emulsion (O/W-Emulsion) enthaltend Polyglyceryl-4 Diisostearate/Polyhydroxystearate/Sebacate in einer Menge von 0,05 bis Gewichts-5 %, bezogen auf das Gesamtgewicht der Zubereitung,
wobei die Zubereitung frei ist von 2-Ethylhexyl-2-cyano-3,3-diphenylacrylat; 4-Methoxyzimtsäure(2-ethylhexyl)ester; 4-Methoxyzimtsäureisoamylester2-Hydroxy-4-methoxybenzophenon; 2-Hydroxy-4-methoxy-4'-methylbenzophenon; 3-(4-Methylbenzyliden)campher und 3-Benzylidencampher, Propyl- und Butyl-Parabenen, Isothiazolinonen, Imidazolidinyl Urea und 3-Iodpropargyl-N-butylcarbamat (IPBC), **dadurch gekennzeichnet, dass** die Emulsion einen oder mehrere UV-Filter gewählt aus der Gruppe der Verbindungen Hexyl 2-[4-(diethylamino)-2-hydroxybenzoyl]benzoate (INCI: Diethylamino hydroxybenzoyl hexyl benzoate), 4-(tert.-Butyl)-4'-methoxydibenzoylmethan, 2,4,6-Tris-[anilino-(p-carbo-2'-ethyl-1'-hexyl-oxy)]-1,3,5-triazin (INCI: Ethylhexyl Triazone), 2,4-Bis-{[4-(2-ethyl-hexyloxy)-2-hydro-xy]-phenyl}-6-(4-methoxyphenyl)-1,3,5-triazin (INCI: Bis- Ethylhexyloxyphenol methoxyphenyl Triazine), 4,4'-[[6-[[4-[[(1,1 dimethylethyl)amino]carbonyl]phenyl] amino]-1,3,5-triazine-2,4-diyl]diimino]bis-, bis(2-ethylhexyl)benzoat (INCI: Diethylhexyl Butamido Triazone), Titandioxid enthält.

25. Kosmetische O/W-Emulsion nach Anspruch 24, **dadurch gekennzeichnet, dass** die Emulsion eine Viskosität von 300 bis 3000 mPas aufweist, gemessen mit Viskosimeter des Typs Rheomat 123 von pro Rheo mit dem Messkörper 1 durchgeführt.

26. Kosmetische O/W-Emulsion nach einem der vorhergehenden Ansprüche 24 bis 25, **dadurch gekennzeichnet, dass** die Emulsion Xanthangummi (Xanthan gum) und/oder Zellulosegummi (Cellulose gum) enthält.

27. Kosmetische O/W-Emulsion nach einem der vorhergehenden Ansprüche 24 bis 26, **dadurch gekennzeichnet, dass** die Emulsion mikrokristalline Zellulose enthält.

28. Kosmetische O/W-Emulsion nach einem der vorhergehenden Ansprüche 24 bis 27, **dadurch gekennzeichnet, dass** die Emulsion einen oder mehrere Emulgatoren gewählt aus der Gruppe der Verbindungen Polyglyceryl-10 stearat, Glycerylstearatcitrat, Glycerylstearat (selbstemulgierend), Polyglyceryl-3-methylglycosedistearat, Natriumcetearylsulfat, Kaliumcetylphosphat, Natriumstearoylglutamat, Sucrosepolystearat enthält.

29. Kosmetische O/W-Emulsion nach einem der vorhergehenden Ansprüche 24 bis 28, **dadurch gekennzeichnet, dass** die Emulsion als Emulgatoren Natriumstearoylglutamat und/oder Sucrosepolystearat enthält.

30. Kosmetische O/W-Emulsion nach einem der vorhergehenden Ansprüche 24 bis 29, **dadurch gekennzeichnet, dass** die Emulsion frei ist von Polyethylenglycol-Ethern und Polyethylenglycol-Estern.

31. Kosmetische O/W-Emulsion nach einem der vorhergehenden Ansprüche 24 bis 30, **dadurch gekennzeichnet, dass** die Emulsion Ethylhexylglycerin enthält.

32. Kosmetische O/W-Emulsion nach einem der vorhergehenden Ansprüche 24 bis 31, **dadurch gekennzeichnet, dass** die Emulsion Vinylpyrrolidon/Hexadecen-Copolymer enthält.

33. Kosmetische O/W-Emulsion nach einem der vorhergehenden Ansprüche 24 bis 32, **dadurch gekennzeichnet, dass** die Emulsion Iminodisuccinat-Salze enthält.

## Claims

1. Cosmetic oil-in-water emulsion (O/W emulsion) comprising polyglyceryl-4 diisostearate/polyhydroxystearate/sebacate in an amount of 0.05% to 5% by weight, based on the total weight of the preparation,
where the preparation is free of 2-ethylhexyl 2-cyano-3,3-diphenylacrylate; 2-ethylhexyl 4-methoxycinnamate; isoamyl 4-methoxycinnamate; 2-hydroxy-4-methoxybenzophenone; 2-hydroxy-4-methoxy-4'-methylbenzophenone; 3-(4-methylbenzylidene)camphor and 3-benzylidenecamphor, propyl- and butylparabens, isothiazolinones, Imidazolidinyl Urea and 3-iodopropargyl N-butylcarbamate (IPBC), **characterized in that** the preparation comprises 2-ethylhexyl 2-hydroxybenzoate (INCI: Ethylhexyl Salicylate) and/or 3,3,5-trimethylcyclohexyl 2-hydroxybenzoate (INCI: Homosalate).

2. Cosmetic O/W emulsion according to Claim 1, **characterized in that** the preparation comprises 2-phenylbenzimidazole-5-sulfonic salts.

3. Cosmetic O/W emulsion according to either of the preceding Claims 1 and 2, **characterized in that** the emulsion comprises one or more UV filters selected from the group of compounds hexyl 2-[4-(diethylamino)-2-hydroxybenzoyl]benzoate (INCI: Diethylamino hydroxybenzoyl hexyl benzoate), 4-(tert-butyl)-4'-methoxydibenzoylmethane, 2,4,6-tris[anilino-(p-carbo-2'-ethyl-1'-hexyloxy)]-1,3,5-triazine (INCI: Ethylhexyl Triazone), 2,4-bis{[4-(2-ethylhexyloxy)-2-hydroxy]phenyl}-6-(4-methoxyphenyl)-1,3,5-triazine (INCI: Bis-Ethylhexyloxyphenol methoxyphenyl Triazine), 4,4'-[[6-[[4-[[(1,1-dimethylethyl)amino]carbonyl]phenyl]amino]-1,3,5-triazine-2,4-diyl]diimino]bis,bis(2-ethylhexyl)benzoate (INCI: Diethylhexyl Butamido Triazone), titanium dioxide.

4. Cosmetic O/W emulsion according to any of the preceding Claims 1 to 3, **characterized in that** the emulsion has a viscosity of 300 to 3000 mPas, measured with the Rheomat 123 type viscometer from pro Rheo with measuring bob 1.

5. Cosmetic O/W emulsion according to any of the preceding Claims 1 to 4, **characterized in that** the emulsion comprises xanthan gum and/or cellulose gum.

6. Cosmetic O/W emulsion according to any of the preceding Claims 1 to 5, **characterized in that** the emulsion comprises microcrystalline cellulose.

7. Cosmetic O/W emulsion according to any of the preceding Claims 1 to 6, **characterized in that** the emulsion comprises one or more emulsifiers selected from the group of compounds polyglyceryl-10 stearate, glyceryl stearate citrate, glyceryl stearate (self-emulsifying), polyglyceryl-3-methylglycose distearate, sodium cetearylsulfate, potassium cetylphosphate, sodium stearoylglutamate, sucrose polystearate.

8. Cosmetic O/W emulsion according to any of the preceding Claims 1 to 7, **characterized in that** the emulsion comprises sodium stearoylglutamate and/or sucrose polystearate as emulsifiers.

9. Cosmetic O/W emulsion according to any of the preceding Claims 1 to 8, **characterized in that** the emulsion is free of polyethylene glycol ethers and polyethylene glycol esters.

10. Cosmetic O/W emulsion according to any of the preceding Claims 1 to 9, **characterized in that** the emulsion comprises ethylhexylglycerol.

11. Cosmetic O/W emulsion according to any of the preceding Claims 1 to 10, **characterized in that** the emulsion comprises vinylpyrrolidone/hexadecene copolymer.

12. Cosmetic O/W emulsion according to any of the preceding Claims 1 to 11, **characterized in that** the emulsion comprises iminodisuccinate salts.

13. Cosmetic oil-in-water emulsion (O/W emulsion) comprising polyglyceryl-4 diisostearate/polyhydroxystearate/sebacate in an amount of 0.05% to 5% by weight, based on the total weight of the preparation,
where the preparation is free of 2-ethylhexyl 2-cyano-3,3-diphenylacrylate; 2-ethylhexyl 4-methoxycinnamate; isoamyl 4-methoxycinnamate; 2-hydroxy-4-methoxybenzophenone; 2-hydroxy-4-methoxy-4'-methylbenzophenone; 3-(4-methylbenzylidene)camphor and 3-benzylidenecamphor, propyl- and butylparabens, isothiazolinones, Imidazolidinyl Urea and 3-iodopropargyl N-butylcarbamate (IPBC), **characterized in that** the preparation comprises 2-phenylbenzimidazole-5-sulfonic salts.

14. Cosmetic O/W emulsion according to Claim 13, **characterized in that** the emulsion comprises one or more UV filters selected from the group of compounds hexyl 2-[4-(diethylamino)-2-hydroxybenzoyl]benzoate (INCI: Diethylamino hydroxybenzoyl hexyl benzoate), 4-(tert-butyl)-4'-methoxydibenzoylmethane, 2,4,6-tris[anilino-(p-carbo-2'-ethyl-1'-hexyloxy)]-1,3,5-triazine (INCI: Ethylhexyl Triazone), 2,4-bis{[4-(2-ethylhexyloxy)-2-hydroxy]phenyl}-6-(4-methoxyphenyl)-1,3,5-triazine (INCI: Bis-Ethylhexyloxyphenol methoxyphenyl Triazine), 4,4'-[[6-[[4-[[(1,1-dimethylethyl)amino]carbonyl]phenyl]amino]-1,3,5-triazine-2,4-diyl]diimino]bis,bis(2-ethylhexyl)benzoate (INCI: Diethylhexyl Butamido Triazone), titanium dioxide.

15. Cosmetic O/W emulsion according to either of the preceding Claims 13 and 14, **characterized in that** the emulsion has a viscosity of 300 to 3000 mPas, measured by Rheomat 123 type viscometer from pro Rheo with measuring bob 1.

16. Cosmetic O/W emulsion according to any of the preceding Claims 13 to 15, **characterized in that** the emulsion comprises xanthan gum and/or cellulose gum.

17. Cosmetic O/W emulsion according to any of the preceding Claims 13 to 16, **characterized in that** the emulsion comprises microcrystalline cellulose.

18. Cosmetic O/W emulsion according to any of the preceding Claims 13 to 17, **characterized in that** the emulsion comprises one or more emulsifiers selected from the group of compounds polyglyceryl-10 stearate, glyceryl stearate citrate, glyceryl stearate (self-emulsifying), polyglyceryl-3-methylglycose distearate, sodium cetearylsulfate, potassium cetylphosphate, sodium stearoylglutamate, sucrose polystearate.

19. Cosmetic O/W emulsion according to any of the preceding Claims 13 to 18, **characterized in that** the emulsion comprises sodium stearoylglutamate and/or sucrose polystearate as emulsifiers.

20. Cosmetic O/W emulsion according to any of the preceding Claims 13 to 19, **characterized in that** the emulsion is free of polyethylene glycol ethers and polyethylene glycol esters.

21. Cosmetic O/W emulsion according to any of the preceding Claims 13 to 20, **characterized in that** the emulsion comprises ethylhexylglycerol.

22. Cosmetic O/W emulsion according to any of the preceding Claims 13 to 21, **characterized in that** the emulsion comprises vinylpyrrolidone/hexadecene copolymer.

23. Cosmetic O/W emulsion according to any of the preceding Claims 13 to 22, **characterized in that** the emulsion comprises iminodisuccinate salts.

24. Cosmetic oil-in-water emulsion (O/W emulsion) comprising polyglyceryl-4 diisostearate/polyhydroxystearate/sebacate in an amount of 0.05% to 5% by weight, based on the total weight of the preparation,
where the preparation is free of 2-ethylhexyl 2-cyano-3,3-diphenylacrylate; 2-ethylhexyl 4-methoxycinnamate; isoamyl 4-methoxycinnamate; 2-hydroxy-4-methoxybenzophenone; 2-hydroxy-4-methoxy-4'-methylbenzophenone; 3-(4-methylbenzylidene)camphor and 3-benzylidenecamphor, propyl- and butylparabens, isothiazolinones, Imidazolidinyl Urea and 3-iodopropargyl N-butylcarbamate (IPBC), **characterized in that** the emulsion comprises one or more UV filters selected from the group of compounds hexyl 2-[4-(diethylamino)-2-hydroxybenzoyl]benzoate (INCI: Diethylamino hydroxybenzoyl hexyl benzoate), 4-(tert-butyl)-4'-methoxydibenzoylmethane, 2,4,6-tris[anilino-(p-carbo-2'-ethyl-1'-hexyloxy)]-1,3,5-triazine (INCI: Ethylhexyl Triazone), 2,4-bis{[4-(2-ethylhexyloxy)-2-hydroxy]phenyl}-6-(4-methoxyphenyl)-1,3,5-triazine (INCI: Bis-Ethylhexyloxyphenol methoxyphenyl Triazine), 4,4'-[[6-[[4-[[(1,1-dimethylethyl)amino]carbonyl]phenyl]amino]-1,3,5-triazine-2,4-diyl]diimino]bis,bis(2-ethylhexyl)benzoate (INCI: Diethylhexyl Butamido Triazone), titanium dioxide.

25. Cosmetic O/W emulsion according to Claim 24, **characterized in that** the emulsion has a viscosity of 300 to 3000 mPas, measured by Rheomat 123 type viscometer from pro Rheo with measuring bob 1.

26. Cosmetic O/W emulsion according to either of the preceding Claims 24 and 25, **characterized in that** the emulsion comprises xanthan gum and/or cellulose gum.

27. Cosmetic O/W emulsion according to any of the preceding Claims 24 to 26, **characterized in that** the emulsion comprises microcrystalline cellulose.

28. Cosmetic O/W emulsion according to any of the preceding Claims 24 to 27, **characterized in that** the emulsion comprises one or more emulsifiers selected from the group of compounds polyglyceryl-10 stearate, glyceryl stearate citrate, glyceryl stearate (self-emulsifying), polyglyceryl-3-methylglycose distearate, sodium cetearylsulfate, potassium cetylphosphate, sodium stearoylglutamate, sucrose polystearate.

29. Cosmetic O/W emulsion according to any of the preceding Claims 24 to 28, **characterized in that** the emulsion comprises sodium stearoylglutamate and/or sucrose polystearate as emulsifiers.

30. Cosmetic O/W emulsion according to any of the preceding Claims 24 to 29, **characterized in that** the emulsion is free of polyethylene glycol ethers and polyethylene glycol esters.

31. Cosmetic O/W emulsion according to any of the preceding Claims 24 to 30, **characterized in that** the emulsion comprises ethylhexylglycerol.

32. Cosmetic O/W emulsion according to any of the preceding Claims 24 to 31, **characterized in that** the emulsion comprises vinylpyrrolidone/hexadecene copolymer.

33. Cosmetic O/W emulsion according to any of the preceding Claims 24 to 32, **characterized in that** the emulsion comprises iminodisuccinate salts.

## Revendications

1. Émulsion huile dans eau cosmétique (émulsion H/E), contenant du diisostéarate/polyhydroxystéarate/sébacate de polyglycéryle-4 en une quantité de 0,05 à 5 % en poids, par rapport au poids total de la préparation,
la préparation étant exempte d'acrylate de 2-éthylhexyl-2-cyano-3,3-diphényle, d'ester 2-éthylhexylique de l'acide 4-méthoxycinnamique, d'ester isoamylique de l'acide 4-méthoxycinnamique, de 2-hydroxy-4-méthoxybenzophénone, de 2-hydroxy-4-méthoxy-4'-méthylbenzophénone, de 3-(4-méthylbenzylidène)camphre et de 3-benzylidène-camphre, de propyl- et butyl-parabènes, d'isothiazolinones, d'imidazolidinyl-urée et de carbamate de 3-iodopropargyl-N-butyle (IPBC), **caractérisée en ce que** la préparation contient du 2-hydroxybenzoate de 2-éthylhexyle (INCI : Ethylhexyl Salicylate) et/ou du 2-hydroxybenzoate de 3,3,5-triméthylcyclohexyle (INCI : Homosalate).

2. Émulsion H/E cosmétique selon la revendication 1, **caractérisée en ce que** la préparation contient des sels de l'acide 2-phénylbenzimidazole-5-sulfonique.

3. Émulsion H/E cosmétique selon l'une quelconque des revendications 1 et 2 précédentes, **caractérisée en ce que** l'émulsion contient un ou plusieurs filtres UV choisis dans le groupe de composés constitué par le 2-[4-(diéthylamino)-2-hydroxybenzoyl]benzoate d'hexyle (INCI : Diethylamino Hydroxybenzoyl Hexyl Benzoate), le 4-(tert.-butyl)-4'-méthoxydibenzoylméthane, la 2,4,6-tris-[anilino-(p-carbo-2'-éthyl-1'-hexyloxy)]-1,3,5-triazine (INCI : Ethylhexyl Triazone), la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazine), le benzoate de 4,4'-[[6-[[4-[[(1,1-diméthyléthyl)amino]carbonyl]phényl]amino]-1,3,5-triazine-2,4-diyl]diimino]bis-,bis(2-éthylhexyle) (INCI : Diethylhexyl Butamido Triazone), le dioxyde de titane.

4. Émulsion H/E cosmétique selon l'une quelconque des revendications 1 à 3 précédentes, **caractérisée en ce que** l'émulsion présente une viscosité de 300 à 3 000 mPas, mesurée avec un viscosimètre de type Rheomat 123 de pro Rheo avec le corps de mesure 1.

5. Émulsion H/E cosmétique selon l'une quelconque des revendications 1 à 4 précédentes, **caractérisée en ce que** l'émulsion contient de la gomme de xanthane (Xanthan gum) et/ou de la gomme de cellulose (Cellulose gum).

6. Émulsion H/E cosmétique selon l'une quelconque des revendications 1 à 5 précédentes, **caractérisée en ce que** l'émulsion contient de la cellulose microcristalline.

7. Émulsion H/E cosmétique selon l'une quelconque des revendications 1 à 6 précédentes, **caractérisée en ce que** l'émulsion contient un ou plusieurs émulsifiants choisis dans le groupe de composés constitué par le stéarate de polyglycéryle-10, le stéarate-citrate de glycéryle, le stéarate de glycéryle (auto-émulsifiant), le distéarate de polyglycéryl-3-méthylglycose, le sulfate de cétéaryle sodique, le phosphate de cétyle potassique, le glutamate de stéaroyle sodique, le polystéarate de sucrose.

8. Émulsion H/E cosmétique selon l'une quelconque des revendications 1 à 7 précédentes, **caractérisée en ce que** l'émulsion contient en tant qu'émulsifiants du glutamate de stéaroyle sodique et/ou du polystéarate de sucrose.

9. Émulsion H/E cosmétique selon l'une quelconque des revendications 1 à 8 précédentes, **caractérisée en ce que** l'émulsion est exempte d'éthers de polyéthylène glycol et d'esters de polyéthylène glycol.

10. Émulsion H/E cosmétique selon l'une quelconque des revendications 1 à 9 précédentes, **caractérisée en ce que** l'émulsion contient de l'éthylhexylglycérine.

11. Émulsion H/E cosmétique selon l'une quelconque des revendications 1 à 10 précédentes, **caractérisée en ce que** l'émulsion contient un copolymère de vinylpyrrolidone/hexadécène.

12. Émulsion H/E cosmétique selon l'une quelconque des revendications 1 à 11 précédentes, **caractérisée en ce que** l'émulsion contient des sels d'iminodisuccinate.

13. Émulsion huile dans eau cosmétique (émulsion H/E), contenant du diisostéarate/polyhydroxystéarate/sébacate de polyglycéryle-4 en une quantité de 0,05 à 5 % en poids, par rapport au poids total de la préparation,
la préparation étant exempte d'acrylate de 2-éthylhexyl-2-cyano-3,3-diphényle, d'ester 2-éthylhexylique de l'acide 4-méthoxycinnamique, d'ester isoamylique de l'acide 4-méthoxycinnamique, de 2-hydroxy-4-méthoxybenzophénone, de 2-hydroxy-4-méthoxy-4'-méthylbenzophénone, de 3-(4-méthylbenzylidène)camphre et de 3-benzylidène-camphre, de propyl- et butyl-parabènes, d'isothiazolinones, d'imidazolidinyl-urée et de carbamate de 3-iodopropargyl-N-butyle (IPBC), **caractérisée en ce que** la préparation contient des sels de l'acide 2-phénylbenzimidazole-5-sulfonique.

14. Émulsion H/E cosmétique selon la revendication 13, **caractérisée en ce que** l'émulsion contient un ou plusieurs filtres UV choisis dans le groupe de composés constitué par le 2-[4-(diéthylamino)-2-hydroxybenzoyl]benzoate d'hexyle (INCI : Diethylamino Hydroxybenzoyl Hexyl Benzoate), le 4-(tert.-butyl)-4'-méthoxydibenzoylméthane, la 2,4,6-tris-[anilino-(p-carbo-2'-éthyl-1'-hexyloxy)]-1,3,5-triazine (INCI : Ethylhexyl Triazone), la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazine), le benzoate de 4,4'-[[6-[[4-[[(1,1-diméthyléthyl)amino]carbonyl]phényl]amino]-1,3,5-triazine-2,4-diyl]diimino]bis-,bis(2-éthylhexyle) (INCI : Diethylhexyl Butamido Triazone), le dioxyde de titane.

15. Émulsion H/E cosmétique selon l'une quelconque des revendications 13 à 14 précédentes, **caractérisée en ce que** l'émulsion présente une viscosité de 300 à 3 000 mPas, mesurée avec un viscosimètre de type Rheomat 123 de pro Rheo avec le corps de mesure 1.

16. Émulsion H/E cosmétique selon l'une quelconque des revendications 13 à 15 précédentes, **caractérisée en ce que** l'émulsion contient de la gomme de xanthane (Xanthan gum) et/ou de la gomme de cellulose (Cellulose gum).

17. Émulsion H/E cosmétique selon l'une quelconque des revendications 13 à 16 précédentes, **caractérisée en ce que** l'émulsion contient de la cellulose microcristalline.

18. Émulsion H/E cosmétique selon l'une quelconque des revendications 13 à 17 précédentes, **caractérisée en ce que** l'émulsion contient un ou plusieurs émulsifiants choisis dans le groupe de composés constitué par le stéarate de polyglycéryle-10, le stéarate-citrate de glycéryle, le stéarate de glycéryle (auto-émulsifiant), le distéarate de polyglycéryl-3-méthylglycose, le sulfate de cétéaryle sodique, le phosphate de cétyle potassique, le glutamate de stéaroyle sodique, le polystéarate de sucrose.

19. Émulsion H/E cosmétique selon l'une quelconque des revendications 13 à 18 précédentes, **caractérisée en ce que** l'émulsion contient en tant qu'émulsifiants du glutamate de stéaroyle sodique et/ou du polystéarate de sucrose.

20. Émulsion H/E cosmétique selon l'une quelconque des revendications 13 à 19 précédentes, **caractérisée en ce que** l'émulsion est exempte d'éthers de polyéthylène glycol et d'esters de polyéthylène glycol.

21. Émulsion H/E cosmétique selon l'une quelconque des revendications 13 à 20 précédentes, **caractérisée en ce que** l'émulsion contient de l'éthylhexylglycérine.

22. Émulsion H/E cosmétique selon l'une quelconque des revendications 13 à 21 précédentes, **caractérisée en ce que** l'émulsion contient un copolymère de vinylpyrrolidone/hexadécène.

23. Émulsion H/E cosmétique selon l'une quelconque des revendications 13 à 22 précédentes, **caractérisée en ce que** l'émulsion contient des sels d'iminodisuccinate.

24. Émulsion huile dans eau cosmétique (émulsion H/E), contenant du diisostéarate/polyhydroxystéarate/sébacate de polyglycéryle-4 en une quantité de 0,05 à 5 % en poids, par rapport au poids total de la préparation,
la préparation étant exempte d'acrylate de 2-éthylhexyl-2-cyano-3,3-diphényle, d'ester 2-éthylhexylique de l'acide 4-méthoxycinnamique, d'ester isoamylique de l'acide 4-méthoxycinnamique, de 2-hydroxy-4-méthoxybenzophénone, de 2-hydroxy-4-méthoxy-4'-méthylbenzophénone, de 3-(4-méthylbenzylidène)camphre et de 3-benzylidène-camphre, de propyl- et butyl-parabènes, d'isothiazolinones, d'imidazolidinyl-urée et de carbamate de 3-iodopropargyl-N-butyle (IPBC), **caractérisée en ce que** l'émulsion contient un ou plusieurs filtres UV choisis dans le groupe de composés constitué par le 2-[4-(diéthylamino)-2-hydroxybenzoyl]benzoate d'hexyle (INCI : Diethylamino Hydroxybenzoyl Hexyl Benzoate), le 4-(tert.-butyl)-4'-méthoxydibenzoylméthane, la 2,4,6-tris-[anilino-(p-carbo-2'-éthyl-1'-hexyloxy)]-1,3,5-triazine (INCI : Ethylhexyl Triazone), la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazine), le benzoate de 4,4'-[[6-[[4-[[(1,1-diméthyléthyl)amino]carbonyl]phényl]amino]-1,3,5-triazine-2,4-diyl]diimino]bis-,bis(2-éthylhexyle) (INCI : Diethylhexyl Butamido Triazone), le dioxyde de titane.

25. Émulsion H/E cosmétique selon la revendication 24, **caractérisée en ce que** l'émulsion présente une viscosité de 300 à 3 000 mPas, mesurée avec un viscosimètre de type Rheomat 123 de pro Rheo avec le corps de mesure 1.

26. Émulsion H/E cosmétique selon l'une quelconque des revendications 24 à 25 précédentes, **caractérisée en ce que** l'émulsion contient de la gomme de xanthane (Xanthan gum) et/ou de la gomme de cellulose (Cellulose gum).

27. Émulsion H/E cosmétique selon l'une quelconque des revendications 24 à 26 précédentes, **caractérisée en ce que** l'émulsion contient de la cellulose microcristalline.

28. Émulsion H/E cosmétique selon l'une quelconque des revendications 24 à 27 précédentes, **caractérisée en ce que** l'émulsion contient un ou plusieurs émulsifiants choisis dans le groupe de composés constitué par le stéarate de polyglycéryle-10, le stéarate-citrate de glycéryle, le stéarate de glycéryle (auto-émulsifiant), le distéarate de polyglycéryl-3-méthylglycose, le sulfate de cétéaryle sodique, le phosphate de cétyle potassique, le glutamate de stéaroyle sodique, le polystéarate de sucrose.

29. Émulsion H/E cosmétique selon l'une quelconque des revendications 24 à 28 précédentes, **caractérisée en ce que** l'émulsion contient en tant qu'émulsifiants du glutamate de stéaroyle sodique et/ou du polystéarate de sucrose.

30. Émulsion H/E cosmétique selon l'une quelconque des revendications 24 à 29 précédentes, **caractérisée en ce que** l'émulsion est exempte d'éthers de polyéthylène glycol et d'esters de polyéthylène glycol.

31. Émulsion H/E cosmétique selon l'une quelconque des revendications 24 à 30 précédentes, **caractérisée en ce que** l'émulsion contient de l'éthylhexylglycérine.

32. Émulsion H/E cosmétique selon l'une quelconque des revendications 24 à 31 précédentes, **caractérisée en ce que** l'émulsion contient un copolymère de vinylpyrrolidone/hexadécène.

33. Émulsion H/E cosmétique selon l'une quelconque des revendications 24 à 32 précédentes, **caractérisée en ce que** l'émulsion contient des sels d'iminodisuccinate.
